# EUROPEAN PATENT APPLICATION

(11) **EP 4 417 694 A1**
(43) Date of publication of application: **21.08.2024**
(21) Application number: 22881396.0
(22) Date of filing: 14.10.2022
(51) Int. Cl.: C12N 15/63, C12N 7/00, A61K 48/00

(54) **GENE CONSTRUCT FOR EXPRESSING MRNA**

(30) Priority: 15.10.2021 KR 20210137521
(71) Applicant: Genomictree, Inc., Daejeon 34027 (KR)
(72) Inventor: AN, Sungwhan, Daejeon 34125 (KR)
(74) Representative: Mewburn Ellis LLP
(86) International application number: PCT/KR2022/015581
(87) International publication number: WO 2023/063769

(57) **Abstract**

The present invention relates to a gene construct for expressing an mRNA, and a pharmaceutical composition, a vaccine composition, and a gene therapy composition, each comprising the gene construct. More specifically, the present invention relates to a gene construct including a coronavirus (SARS-CoV-2: Severe acute respiratory syndrome coronavirus 2)-derived 5' untranslated region (UTR) and/or 3' untranslated region (UTR), and a pharmaceutical composition, a vaccine composition, and a gene therapy composition, each comprising the gene construct.

## Description

### [Technical Field]

The present invention relates to a gene construct for expressing mRNA, and a pharmaceutical composition, a vaccine composition, and a gene therapy composition, each containing the gene construct, and more specifically, to a gene construct including a coronavirus-derived 5' untranslated region (UTR) and/or 3' untranslated region (UTR), and a pharmaceutical composition, a vaccine composition, and a gene therapy composition, each containing the gene construct.

### [Background Art]

Gene therapy has been developed as therapeutics that treat or prevent diseases by adding new information to existing genes, modifying the genes with the information or deleting the genes, and delivering the resulting genes to the cells of patients in the form of plasmid DNA (pDNA) or mRNA (messenger RNA) (Dunbar, C. E et al., Science 2018 1:12).

The gene therapy aims to cure diseases by correcting genetic defects in patients who suffer from genetic diseases or rare diseases. Recent technological innovations and investments in research and development have brought about growth of the global gene therapy market at an average annual rate of 10%, and gene therapy is emerging as a new blue chip in the pharmaceutical market. Thereamong, mRNA is emerging as a promising therapeutic means in the field of vaccine development and protein replacement therapy. Gene therapy is based on the principle in which genes are introduced into cells in the form of pDNA or mRNA using viruses, liposomes, anti-sense technology, and the like to allow the genes to act as therapeutics (Dunbar, C. E et al., Science 2018 1:12).

mRNA therapeutics are advantageously superior to DNA or virus therapeutics in terms of stability, efficiency, and productivity. First, mRNA occurs in the cytoplasm and thus has a low probability of mutation due to infection or genomic DNA insertion. Second, mRNA may be modified in various forms within the cytoplasm and thus can improve intracellular stability by controlling the half-life or increasing the amount of translated protein. Third, mRNA is suitable for *in vitro* experiments and thus causes rapid development and GMP production and is advantageously suitable for massproduction (Wang, Y., Su et al. Molecular therapy 2013 358-367) .

mRNA refers to RNA that transmits the genetic information of DNA to ribosomes and causes protein expression through translation by mRNA. Recombinant mRNA for developing therapeutic agents or vaccines is produced from linear DNA using a promoter such as T7 or SP6 (but is not limited thereto) and an RNA polymerase, and is then 5' capped and poly A adenylated (poly A tailed) by *in vitro* transcription. The mRNA thus produced has a structure similar to the mature mRNA in the cytoplasm and consists of 5' capping, 5' UTR (untranslated region), 3' UTR, poly A, and a target gene to be expressed. Thereamong, the untranslated region (5' or 3' untranslated region) affects the stability and translational activation of mRNA depending on the sequence, thus increasing the half-life and expression level. In addition, Poly A prevents the degradation of mRNA in the cytoplasm, increasing stability and expression level. Therefore, in order for mRNA therapeutics to efficiently and stably express proteins in the body, it is important to find the optimal untranslated region sequence and poly A length (Pardi, N et al,. Nature reviews 2018 261-279).

Under this technical background, the preset inventors developed a gene construct including a 5' untranslated region (UTR) and/or a 3' untranslated region (UTR) derived from coronavirus (SARS-CoV-2). Based thereon, the present invention has been completed.

### [Disclosure]

Therefore, the present invention has been made in view of the above problems, and it is one object of the present invention to provide a gene construct including a 5' untranslated region (UTR) and/or 3' untranslated region (UTR) derived from coronavirus.

It is another object of the present invention to provide a vector including the gene construct.

It is another object of the present invention to provide a pharmaceutical composition containing the gene construct.

It is another object of the present invention to provide a vaccine composition containing the gene construct.

It is another object of the present invention to provide a composition for gene therapy containing the gene construct.

In accordance with one aspect of the present invention, the above and other objects can be accomplished by the provision of a gene construct including a coding region for expressing mRNA of a target gene, a 5' untranslated region (UTR) located upstream of the coding region, and a 3' untranslated region (UTR) located downstream of the coding region, wherein the 5' untranslated region (UTR) includes a leader sequence of a coronavirus.

In accordance with another aspect of the present invention, provided is a vector including the gene construct.

It is another object of the present invention to provide a pharmaceutical composition containing the gene construct. It is another object of the present invention to provide a vaccine composition containing the gene construct.

It is another object of the present invention to provide a composition for gene therapy containing the gene construct.

### [Description of Drawings]

FIG. 1 is a schematic diagram illustrating the coronavirus structure and SARS-CoV-2 genomic and subgenomic RNA expression, wherein (A) shows the appearance of the coronavirus and the amount of mRNA for each protein analyzed by Northern blot and (B) shows the genomic RNA structure of SARS-CoV-2 and representative subgenomic RNAs expressed therefrom.
FIG. 2 is a schematic diagram illustrating template DNA for expressing mRNA.
FIG. 3 illustrates a process of producing template DNA by PCR to express mRNA of EGFP genes.
FIG. 4 illustrates a process of producing template DNA by PCR to express mRNA of SARS-CoV-2 N genes.
FIG. 5 illustrates a process of producing template DNA by PCR to express mRNA of human ADCYAP1 genes.
FIG. 6 illustrates mRNA expression of control, EGFP, ADCYAP1, and N genes identified using IVT (*in vitro* transcription).
FIG. 7 illustrates expression of GFP protein in the HeLa cell line transfected with the GFP mRNA produced in FIG. 6, identified by images and Western blotting.
FIG. 8 illustrates inhibition of growth of cervical cancer cells by the ADCYAP1 protein expressed in the HeLa cell line transfected with the ADCYAP1 gene mRNA prepared in FIG. 6, and identification thereof using Western blotting.
FIG. 9 illustrates inhibition of growth of cervical cancer cells by the ADCYAP1 protein expressed in the HeLa cell line transfected with the N gene mRNA prepared in FIG. 6, and identification thereof using Western blotting.

### [Best Mode]

Unless defined otherwise, all technical and scientific terms used herein have the same meanings as appreciated by those skilled in the field to which the present invention pertains. In general, the nomenclature used herein is well-known in the art and is ordinarily used.

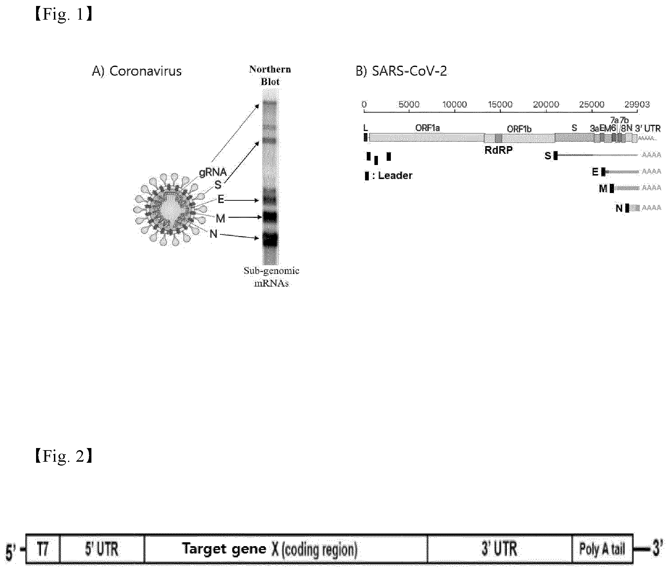

COVID-19, which has recently become prevalent around the world, is caused by infection with SARS-CoV-2, a variant of the coronavirus. Coronavirus has a configuration in which about 30 kb of (+) RNA surrounded by the N (nucleocapsid) protein is present as the genome, M (membrane) and E (envelope) proteins constitute a membrane along with a lipid on the genome, and a spike protein protrudes outward to form a crown-like appearance (FIG. 1).

The coronavirus genome (genomic RNA) enters the cells after infection and immediately acts as mRNA, and transcription occurs in orf1b (open reading frame 1b) located at the front top, producing RdRP (RNA dependent RNA polymerase). RdRP is an enzyme of synthesizing RNA using RNA as a template (Imbert et al., EMBO Journal, 2006, 25:4933-4942).

Therefore, RdRP synthesizes negative strand (-) genomic RNA with the same length by replication using genomic RNA that enters the cell as a template. The (-) genomic RNA thus produced acts as a template for transcription to produce various mRNAs for producing various proteins.

Although the transcriptional mechanism model of coronavirus has not yet been clearly established, subgenomic RNAs acting as mRNAs required to produce essential proteins (S, E, M, N) are known to be present in cells taking into consideration the virus life cycle after infection (Krzysztof Pyre et al., Virology Journal 2004, 1:7).

Regarding the structural characteristics, all subgenomic RNAs have different lengths, but have the rear regions (3' end regions, 3'-UTR regions) common to the genomic RNA.

In addition, a leader sequence including a short base sequence (about 75 nucleotides) is present at the front (5' end) of the genomic RNA, which is also added to the front of all subgenomic RNAs. All subgenomic RNAs have an intergenic sequence between the coding sequence for producing proteins and the leader sequence, the leader sequence and the intergenic sequence act as the 5' UTR that can be recognized by ribosomes during protein expression, and all subgenomic RNAs include a 3' UTR sequence at the 3' end thereof.

Therefore, the desired proteins can be expressed by linking the SARS-CoV-2 leader sequence and the intergenic sequence acting as the 5' UTR, and linking the coding sequence of the gene to be expressed right behind, and the 3' UTR sequence of SARS-CoV-2, and the polyA sequence to produce mRNAs and then introducing the mRNAs into calls (FIG.1). In addition, the leader sequences derived from human coronaviruses 229E, OC43, HKU1, NL63, SARS-1, and MERS may also be used as 5' UTRs.

In this case, the mRNA of the target gene may be produced by IVT (*in vitro* transcription) by linking a promoter sequence (such as T7 or SP7) to the 5' end of the sequence in order to express the mRNA of the desired target gene *in vitro.*

In one aspect, the present invention is directed to a gene construct including a coding region for expressing mRNA of a target gene, a 5' untranslated region (UTR) located upstream of the coding region, and a 3' untranslated region (UTR) located downstream of the coding region, wherein the 5' untranslated region (UTR) includes a leader sequence of a coronavirus.

Regarding the leader sequence, genomic RNA and transcribed subgenomic mRNAs in the coronavirus have in common a leader sequence including approximately 72 to 77 bp at the 5' end, and the leader sequence is a unique characteristic of coronaviruses and is the most abundant target in infected cells among viral sequences because all subgenomic RNAs of coronaviruses have a phenomenon of leader joining in which a leader sequence of approximately 72 bp derived from the 5' end of the genomic RNA binds to the 5' end of each subgenomic RNA. Therefore, the leader sequence has the highest copy number among the viral genes in the cells and the subgenomic RNA encoding N proteins has the next highest copy number.

Specifically, the 5' untranslated region (UTR) includes a leader sequence of the coronavirus (SARS-CoV-2: severe acute respiratory syndrome coronavirus 2) represented by SEQ ID NO: 1.

The leader sequence includes a sequence represented by SEQ ID NO: 1 (ATTAAAGGTTTATACCTTCCCAGGTAACAAACCAACCAACTTTCGATCTCTTGTAGA TCTGTTCTCTAAACG) .

As used herein, the term "nucleic acid" preferably means DNA or RNA. The terms relating to the nucleic acid, such as "polynucleotide," "nucleotide," "nucleotide sequence," and "oligonucleotide", are used interchangeably. The nucleic acid may include polymeric forms of nucleotides with any length, deoxyribonucleotides or ribonucleotides, or analogs thereof. Polynucleotides may have any three-dimensional structure and perform any known or unknown functions. A polynucleotide may include one or more modified nucleotides such as methylated nucleotides and nucleotide analogs. Modifications of the nucleotide structure may be possible before or after assembly of polymers.

Preferably, the nucleic acid is a polymer that contains or consists of nucleotide monomers covalently linked to each other by phosphodiester bonds of the sugar/phosphate backbone. The nucleic acid includes modified nucleic acids such as base-modified, sugarmodified or backbone-modified DNA or RNA molecules.

The DNA is an abbreviation for deoxyribonucleic acid and is a nucleic acid molecule, namely, a polymer composed of nucleotides. These nucleotides are usually deoxyadenosine-monophosphate, deoxy-thymidine-monophosphate, deoxy-guanosine-monophosphate and deoxy-cytidine-monophosphate monomers, contain sugars (deoxyribose), bases and phosphates, and are polymerized by the unique backbone structure thereof. The backbone structure is typically formed by a phosphodiester bond between the sugar moiety of one nucleotide, i.e. deoxyribose, and the phosphate moiety of the adjacent monomer. The specific sequence of monomers, i.e. the sequence of bases linked to the sugar/phosphate backbone, is called "DNA sequence". DNA may be single stranded or double stranded. In the double-stranded form, nucleotides of the first strand typically hybridize with nucleotides of the second strand, for example by A/T base pairing and G/C base pairing.

The RNA includes, for example, mRNA. RNA is an abbreviation for ribonucleic acid. RNA is a nucleic acid molecule, a polymer composed of nucleotides. Nucleotides are usually adenosine-monophosphate, uridine-monophosphate, guanosine-monophosphate and cytidine-monophosphate monomers linked to each other through a so-called "backbone". The backbone is formed by a phosphodiester bond between the sugar, i.e., ribose, and the phosphate moiety of the adjacent monomer. A specific consecutive sequence of monomers is called an "RNA sequence". Usually, RNA may be obtained, for example, within cells, by transcription of a DNA sequence. In eukaryotic cells, transcription is typically performed within the nucleus or mitochondria. *In vivo,* mRNA, messenger RNA, is transcribed from DNA. For example, processing of RNA in eukaryotic cells involves a variety of other post-transcriptional modifications such as splicing, 5'-capping, polyadenylation, and exports from the nucleus or mitochondria and the like. Messenger RNA usually provides a nucleotide sequence that may be translated into the amino acid sequence of a specific peptide or protein. Typically, mRNA includes a 5'-cap, a 5'-UTR, an open reading frame, a 3'-UTR and a poly(A) sequence. Aside from messenger RNA, there are several non-coding forms of RNA that may be involved in the regulation of transcription and/or translation.

The 5'-UTR is located at the 5' end (i.e., "upstream") of an open reading frame. The 5'-UTR starts at the transcription start site and ends at the nucleotide before the start codon of the open reading frame. The 5'UTR may contain elements that regulate gene expression, for example ribosome-binding sites. The 5'UTR may be modified post-transcriptionally, for example by addition of a 5'-cap. 5'UTR corresponds to the sequence of the mature mRNA located between the 5'-cap and the start codon.

The 5' untranslated region (UTR) is formed by linking the SARS-CoV-2 leader sequence to the intergenic sequence, and may include one or more sequences selected from the group consisting of SEQ ID NO: 2 to SEQ ID NO: 4.

The 3'UTR is typically a portion of mRNA located between the protein-coding region of the mRNA (i.e. the open reading frame) and the poly(A) sequence. The 3'UTR of mRNA is not translated into amino acid sequence. 3'UTR sequences are usually encoded by genes, which are each transcribed into mRNA during the gene expression process. The genomic sequence is first transcribed into mRNA containing an optional intron. The mRNA then undergoes steps such as 5' capping, splicing and modification of the 3'-end, such as polyadenylation of the 3'-end, and optional endo- or exonuclease digestion. The 3'UTR is located immediately next to the stop codon 3' of the protein coding region and includes the nucleotide immediately next to the 5' poly(A) sequence.

The 3' untranslated region (UTR) may include the 3' UTR sequence of SARS-CoV-2 and may include the sequence of SEQ ID NO: 5.

The 3' untranslated region (UTR) typically has a sequence of several nucleotide triplets that may be translated into a peptide or protein. The open reading frame preferably includes a start codon, i.e. a combination of three subsequent nucleotides, usually encoding the amino acid methionine (ATG or AUG) at the 5'-end thereof, and a subsequent region usually having a length that is a multiple of 3 nucleotides. The ORF is preferably terminated by a stop codon (e.g., TAA, TAG, TGA). This is the only stop codon in the open reading frame. Therefore, in the context of the present invention, the open reading frame preferably starts with a start codon (e.g., ATG or AUG) and preferably stops with a stop codon (e.g., TAA, TGA, or TAG or UAA, UAG, UGA, respectively), and is a nucleotide sequence including three nucleotides. The open reading frame may be isolated or incorporated into longer nucleic acid sequences, such as vectors or mRNAs. The open reading frame may also be called a "polypeptide or protein coding region".

According to the present invention, the gene construct may further include a promoter and/or poly(A) sequence.

The promoter may be located upstream of the 5' untranslated region (UTR). The promoter may include elements necessary for transcription, such as an RNA polymerase promoter. The promoter may include a phage RNA polymerase promoter such as SP6 or T7, preferably a T7 promoter encoding an mRNA sequence.

The length of the poly (A) sequence may vary. For example, the poly(A) sequence may be about 20 adenine nucleotides to about 300 adenine nucleotides, preferably about 40 to about 200 adenine nucleotides, such as 60, 70, 80, 90 or 100 adenine nucleotides, more preferably about 50 to about 100 adenine nucleotides, or about 20 adenine nucleotides to about 400 adenine nucleotides.

The poly(A) sequence may be located downstream of the 3' untranslated region (UTR). For example, the poly(A) sequence may be linked directly or through a linker, for example, through a linker of 1 to 50 nucleotides, preferably 1 to 20 nucleotides, or through a stretch of 2, 4, 6, 8, 10, and 20 nucleotides.

In a specific embodiment, the gene construct according to the present invention may include a structure of 5'-promoter-5'UTR-coding region (ORF)-3'UTR-poly(A) in the 5'- to -3' direction. Specifically, template DNA including the sequence of 5'-T7 promoter-5'UTR-target gene-3' UTR-poly A may be produced to express the mRNA of the target gene by *in vitro* transcription (IVT) using the 5'UTR and 3'UTR sequences of SARS-CoV-2.

In another aspect, the present invention is directed to a vector containing the gene construct. The vector may be an expression vector, cloning vector, or the like. The vector may be used to produce expression products such as mRNA or peptides, polypeptides or proteins. The vector may include a sequence required for transcription of the sequence stretch of the vector, such as the promotor sequence, for example, an RNA promoter sequence. The vector may be, for example, an RNA vector or a DNA vector. The vector may be a viral vector or a plasmid vector.

The vector may be a circular molecule and the vector may include a double-stranded molecule. Circular, double-stranded DNA molecules may be readily used as a storage form in innovative artificial nucleic acid molecules. The vector may be used for transformation of cells, for example, cultured cells. The vector may be used for *in vitro* transcription to obtain artificial RNA molecules. The circular vector may be linearized, for example, by restriction enzyme digestion.

In some cases, the vector may include sequences suitable for a cloning site, a selection marker such as an antibiotic resistance factor, and amplification of the vector, such as an origin of replication.

The vector is suitable for transcription using eukaryotic, prokaryotic, or eukaryotic, prokaryotic, viral, or phage transcription systems, such as eukaryotic, prokaryotic, viral, or phage *in vitro* transcription systems. In some cases, the vector is suitable for *in vitro* transcription using an *in vitro* transcription system-based phage, such as *in vitro* transcription system-based T7 RNA polymerase.

The vector may be transferred to cells using various methods known in the art such as microinjection, electroporation, DEAE-dextran treatment, lipofection, nanoparticle-mediated transfection, protein transduction domain-mediated transfection, and PEG-mediated transfection, but are not limited thereto.

Known expression vectors such as plasmid vectors, cosmid vectors, and bacteriophage vectors may be used as the vectors, and the vectors may be easily prepared by those skilled in the art depending on any known method using DNA recombination technology.

A recombinant expression vector may contain a nucleic acid in a form suitable for expression of the nucleic acid a host cells, which means that the recombinant expression vector contains at least one regulatory element that may be selected based on the host cell such that the recombinant expression vector is used for expression, namely, the regulatory element is operably linked to the nucleic acid sequence to be expressed.

The term "operably linked" in the recombinant expression vector means that the nucleotide sequence of interest is linked to the regulatory element in a manner allowing expression of the nucleotide sequence (e.g., in an *in vitro* transcription/translation system or in a host cell when the vector is introduced into the host cell).

The recombinant expression vector may contain a form suitable for messenger RNA synthesis, including the T7 promoter, which means that the recombinant expression vector contains at least one regulatory element that allows for *in situ* mRNA synthesis, i.e., allows messenger RNA to be synthesized by T7 polymerase.

The regulatory element may include promoters, enhancers, internal ribosome entry sites (IRES), and other expression regulatory elements (e.g., transcription termination signals such as polyadenylation signals and poly-U sequences). The regulatory element may include elements that cause the induction or constitutive expression of a nucleotide sequence in many types of host cells and elements that cause expression of a nucleotide sequence only in specific host cells (e.g., tissue-specific regulatory sequences) .

Specifically, the regulatory element may include a gene construct including the sequence of SEQ ID NO: 18 or 19.

The nucleic acid may be injected in the form of ribonucleic acid, for example, messenger ribonucleic acid (mRNA). The nucleic acid according to the present invention may be mRNA. This allows for transient protein expression.

In another aspect, the present invention is directed to a vaccine composition containing the gene construct.

In another aspect, the present invention is directed to a composition for gene therapy containing the gene construct.

The composition may include a delivery means for delivering the mRNA expressed in the composition.

The expressed mRNA may be delivered via nanoparticles. For example, the expressed mRNA may be delivered via gold nanoparticles.

The surface of the gold nanoparticle may be modified. The modification may be exemplified in detail in Acc Chem Res. 2019 June 18; 52(6): 1496-1506. and Pharmaceutics 2021, 13, 900., etc., which are incorporated herein by reference.

Gold nanoparticles may be linked to the mRNA to form a complex with a cationic endosomal disruptive polymer and the complex may be delivered to cells (Nature Biomedical Engineering volume 1, pages889-901 (2017)). The cationic endosomal disruptive polymer is, for example, polyethylene imine, poly(arginine), poly(lysine), poly(histidine), poly-[2-{(2-aminoethyl)amino}-ethyl-aspartamide] (pAsp(DET)), a block co-polymer of poly(ethylene glycol) (PEG) and poly(arginine), a block co-polymer of PEG and poly(lysine), or a block co-polymer of PEG and poly{N-[N-(2-aminoethyl)-2-aminoethyl]aspartamide} (PEG-pAsp(DET)).

In some cases, gold particles surface-modified with arginine may be used.

Gold particles modified with arginine may be assembled with a nuclease or a polynucleotide encoding the same and/or a cleavage factor, or a polynucleotide encoding the same. As a result, the result fuses with the membrane of the target cell and then moves into the cytoplasm (ACS Nano. 2017, 11:2452-2458).

Expressed mRNA may be delivered through liposomes, lipid nano-particles (LNPs), or various nanoparticles. Liposomes or LNPs contain cationic lipids, non-cationic lipids, or neutral lipids, and may further contain other lipids such as PEG (polyethylene glycol) or cholesterol. These mRNA delivery systems are described in detail in U.S. Patent Publication Nos. 2018/0311176, 2019/0032051, and 2021/0046192, International Patent Publication Nos. WO 2018/081480, WO 2020/097540, WO 2020/097548, and WO 2021/007278, and the like, which are incorporated herein by reference.

The cationic lipid is exemplified in detail in US Patent Publication Nos. 2018/0311176 and 2019/0032051, and the like, and is, for example, N,N-dioleyl-N,N-dimethylammonium chloride (DODAC), N,N-distearyl-N,N-dimethylammonium bromide (DDAB), N-(I-(2,3-dioleyloxy)propyl)-N,N,N-trimethylammonium chloride (DOTAP), N-(I-(2,3-dioleyloxy)propyl)-N,N,N-trimethylammonium chloride (DOTMA), N,N-dimethyl-2,3-dioleyloxy)propylamine (DODMA), 1,2-dilinoleyloxy-N,N-dimethylaminopropane (DLinDMA), 1,2-dilinolenyloxy-N,N-dimethylaminopropane (DLenDMA), 1,2-dilinoleylcarbamoyloxy-3-dimethylaminopropane (DLin-C-DAP), 1,2-dilinoleyoxy-3-(dimethylamino)acetoxypropane (DLin-DAC), 1,2-dilinoleyoxy-3-morpholinopropane (DLin-MA), 1,2-dilinoleoyl-3-dimethylaminopropane (DLinDAP), 1,2-dilinoleylthio-3-dimethylaminopropane (DLin-S-DMA), 1-linoleoyl-2-linoleyloxy-3-dimethylaminopropane (DLin-2-DMAP), 1,2-dilinoleyloxy-3-trimethylaminopropane chloride salt (DLin-TMA·Cl), 1,2-dilinoleoyl-3-trimethylaminopropane chloride salt (DLin-TAP·Cl), 1,2-dilinoleyloxy-3-(N-methylpiperazino)propane (DLin-MPZ), or 3-(N,N-dilinoleylamino)-1,2-propanediol (DLinAP), 3-(N,N-dioleylamino)-1,2-propanediol (DOAP), 1,2-dilinoleyloxo-3-(2-N,N-dimethylamino)ethoxypropane (DLin-EG-DMA), 1,2-dilinolenyloxy-N,N-dimethylaminopropane (DLinDMA), 2,2-dilinoleyl-4-dimethylaminomethyl-[1,3]-dioxolane (DLin-K-DMA) or analogs thereof, (3aR,5s,6aS)-N,N-dimethyl-2,2-di((9Z,12Z)-octadeca-9,12-dienyl)tetrahydro-3aH-cyclopenta[d][1,3]dioxol-5-amine (ALN100), (6Z,9Z,28Z,31Z)-heptatriaconta-6,9,28,31-tetraen-19-yl 4-(dimethylamino)butanoate (MC3), 1,1'-(2-(4-(2-((2-(bis(2-hydroxydodecyl)amino)ethyl)(2-hydroxydodecyl)amino)ethyl)piperazin-1-yl)ethylazanediyl)didodecan-2-ol (Tech G1), 2,2-dilinoleyl-4-dimethylaminoethyl-[1,3]-dioxolane, β-L-arginyl-2,3-L-diaminopropionic acid-N-palmityl-N-oleylamide trihydrochloride, N',N'-dioctadecyl-N-4,8-diaza-10-aminodecanoylglycine amide [71], 1,2-dilinoleyloxy-3-dimethylaminopropane, DLin-KC2-DMA, amino lipid 2,2-dilinoleyl-4-dimethylaminoethyl-[1,3]-dioxolane (DLin-KC2-DMA, 1), 1,2-distearloxy-/V,N-dimethylaminopropane (DSDMA), dilinoleylmethyl-4-dimethylaminobutyrate (DLin-MC3-DMA), DLin-D-DMA, C12-200, 98N12-5, (20Z,23Z)-N,N-dimethylnonacosa-20,23-dien-10-amine, (17Z,20Z)-N,N-dimemylhexacosa-17,20-dien-9-amine, (1Z,19Z)-N5N-dimethylpentacosa-16,19-dien-8-amine, (13Z,16Z)-N,N-dimethyldocosa-13,16-dien-5-amine, (12Z,15Z)-N,N-dimethylhenicosa-12,15-dien-4-amine, (14Z,17Z)-N,N-dimethyltricosa-14,17-dien-6-amine, (15Z,18Z)-N,N-dimethyltetracosa-15,18-dien-7-amine, (18Z,21Z)-N,N-dimethylheptacosa-18,21-dien-10-amine, (15Z,18Z)-N,N-dimethyltetracosa-15,18-dien-5-amine, (14Z,17Z)-N,N-dimethyltricosa-14,17-dien-4-amine, (19Z,22Z)-N,N-dimethyloctacosa-19,22-dien-9-amine, (18Z,21Z)-N,N-dimethylheptacosa-18,21-dien-8-amine, (17Z,20Z)-N,N-dimethylhexacosa-17,20-dien-7-amine, (16Z,19Z)-N,N-dimethylpentacosa-16,19-dien-6-amine, (22Z,25Z)-N,N-dimethylhentriaconta-22,25-dien-10-amine, (21Z,24Z)-N,N-dimethyltriaconta-21,24-dien-9-amine, (18Z)-N,N-dimethylheptacos-18-en-10-amine, (17Z)-N,N-dimethylhexacos-17-en-9-amine, (19Z,22Z)-N,N-dimethyloctacosa-19,22-dien-7-amine, N,N-dimethylheptacosan-10-amine, (20Z,23Z)-N-ethyl-N-methylnonacosa-20,23-dien-10-amine, 1-[(11Z,14Z)-1-nonylicosa-11,14-dien-1-yl]pyrrolidine, (20Z)-N,N-dimethylheptacos-20-en-10-amine, (15Z)-N,N-dimethyleptacos-15-en-10-amine, (14Z)-N,N-dimethylnonacos-14-en-10-amine, (17Z)-N,N-dimethylnonacos-17-en-10-amine, (24Z)-N,N-dimethyltritriacont-24-en-10-amine, (20Z)-N,N-dimethylnonacos-20-en-10-amine, (22Z)-N,N-dimethylhentriacont-22-en-10-amine, (16Z)-N,N-dimethylpentacos-16-en-8-amine, (12Z,15Z)-N,N-dimethyl-2-nonylhenicosa-12,15-dien-1-amine, (13Z,16Z)-N,N-dimethyl-3-nonyldocosa-13,16-dien-1-amine, N,N-dimethyl-1-[(1S,2R)-2-octylcyclopropyl]heptadecan-8-amine, 1-[(1S,2R)-2-hexylcyclopropyl]-N,N-dimethylnonadecan-10-amine, N,N-dimethyl-1-[(1S,2R)-2-octylcyclopropyl]nonadecan-10-amine, N,N-dimethyl-21-[(1S,2R)-2-octylcyclopropyl]henicosan-10-amine,N,N-dimeth-yl-1-[(1S,2S)-2-{[(1R,2R)-2-pentylcyclopropyl]methyl}cyclopropyl]nonadecan-10-amine,N,N-dimethyl-1-[(1S,2R)-2-octylcyclopropyl]hexadecan-8-amine, N,N-dimethyl-[(1R,2S)-2-undecylcyclopropyl]tetradecan-5-amine, N,N-dimethyl-3-{7-[(1S,2R)-2-octylcyclopropyl]heptyl}dodecan-1-amine, 1-[(1R,2S)-2-heptylcyclopropyl]-N,N-dimethyloctadecan-9-amine, 1-[(1S,2R)-2-decylcyclopropyl]-N,N-dimethylpentadecan-6-amine, N,N-dimethyl-1-[(1S,2R)-2-octylcyclopropyl]pentadecan-8-amine, R-N,N-dimethyl-1-[(9Z,12Z)-octadeca-9,12-dien-1-yloxy]-3-(octyloxy)propa-n-2-amine, S-N,N-dimethyl-1-[(9Z,12Z)-octadeca-9,12-dien-1-yloxy]-3-(octy-loxy)propan-2-amine, 1-{2-[(9Z,12Z)-octadeca-9,12-dien-1-yloxyJ-1-Roctyloxy)methyl]ethyl}pyrro-lidine, (2S)-N,N-dimethyl-1-[(9Z,12Z)-octadeca-9,12-dien-1-yloxy]-3-[(5Z)-oct-5-en-1-yloxy]propan-2-amine, 1-{2-[(9Z,12Z)-octadeca-9,12-dien-1-yloxy]-1-[(octyloxy)methyl]ethyl}azet-idine, (2S)-1-(hexyloxy)-N,N-dimethyl-3-[(9Z,12Z)-octadeca-9,12-dien-ylo-xy]propan-2-amine, (2S)-1-(heptyloxy)-N,N-dimethyl-3-[(9Z,12Z)-octadeca-9,12-dien-1-yloxylpr-opan-2-amine, N,N-dimethyl-1-(nonyloxy)-3-[(9Z,12Z)-octadeca-9,12-dien-1-yloxy]propan-2-amine, N,N-dimethyl-1-[(9Z)-octadec-9-en-1-yloxy]-3-(octyloxy)propan-2-amine, (2S)-N,N-dimethyl-1-[(6Z,9Z,12Z)-octadeca-6,9,12-trien-1-yloxy]-3-(o-ctyloxy)propan-2-amine, (2S)-1-[(11Z,14Z)-icosa-11,14-dien-1-yloxy]-N,N-dimethyl-3-(pentyloxy)propa-n-2-amine, (2S)-1-(hexyloxy)-3-[(11Z,14Z)-icosa-11,14-dien-1-yloxy]-N,N-di-methylpropan-2-amine, 1-[(11Z,14Z)-icosa-11,14-dien-1-yloxy]-N,N-dimethyl-3-(octyloxy)propan-2-amine, 1-[(13Z,16Z)-docosa-13,16-dien-1-yloxy]-N,N-dimethyl-3-(octyloxy)pr-opan-2-amine, (2S)-1-[(13Z,16Z)-docosa-13,16-dien-1-yloxy]-3-(hexyloxy)-N,N-dimethylpro-pan-2-amine, (2S)-1-[(13Z)-docos-13-en-1-yloxy]-3-(hexyloxy)-N,N-dimethylpropan-2-amin-e, 1-[(13Z)-docos-13-en-1-yloxy]-N,N-dimethyl-3-(octyloxy)propan-2-amine, 1-[(9Z)-hexadec-9-en-1-yloxy]-N,N-dimethyl-3-(octyloxy)propan-2-amine, (2R)-N,N-dimethyl-H(1-metoyloctyl)oxy]-3-[(9Z,12Z)-octadeca-9,12-dien-1-yloxy]propan-2-amine, (2R)-1-[(3,7-dimethyloctyl)oxy]-N,N-dimethyl-3-[(9Z,12Z)-octadeca-9,12-di-en-1-yloxy]propan-2-amine, N,N-dimethyl-1-(octyloxy)-3-({8-[(1S,2S)-2-{[(1R,2R)-2-pentylcyclopropyl]-methyl}cyclopropyl]octyl}oxy)propan-2-amine, N,N-dimethyl-1-{[8-(2-oclylcyclopropyl)octyl]oxy}-3-(octyloxy)propan-2-am-ine and (11E,20Z,23Z)-N,N-dimethylnonacosa-11,20,2-trien-10-amine, 5-carboxyspermylglycine dioctaoleoylamide ("DOGS"), dipalmitoylphosphatidylethanolamine 5-carboxyspermyl-amide ("DPPES"), 1,2-dimyristyloxypropyl-3-dimethyl-hydroxy ethyl ammonium bromide (DMRIE), DMRIE-HP, Lipofectamine (DOSPA), 3b-(N-(N',N'-dimethylaminoethane)-carbamoyl)cholesterol ("DC-Choi"), N-(1,2-dimyristyloxyprop-3-yl)-N,N-dimethyl-N-hydroxyethyl ammonium bromide ("DMRIE"), 1,2-dioleoyl-3-dimethylammonium-propane ("DODAP"), DMDMA, cationic lipidbased transfection reagents TransIT-TKO, LIPOFECTIN, Lipofectamine, OLIGOFECTAMINE or DHARMAFECT, DSDMA, DODMA, DLinDMA, DLenDMA, gamma-DLenDMA, DLin-K-DMA, DLin-K-C2-DMA (also known as DLin-C2K-DMA, XTC2, and C2K), DLin-K-C3-DM A, DLin-K-C4-DMA, DLen-C2K-DMA, y-DLen-C2K-DMA, DLin-M-C2-DMA (also known as MC2), DLin-M-C3-DMA (also known as MC3) or (DLin-MP-DMA) (also known as 1-B11), or a mixture thereof, but is not limited thereto.

The non-cationic lipid is exemplified in detail in US Patent Publication Nos. 2018/0311176 and 2019/0032051, and is, for example, phosphatidylglycerol, cardiolipin, diacylphosphatidylserine, diacylphosphatidic acid, N-dodecanoyl phosphatidylethanolamine, N-succinyl phosphatidylethanolamine, N-glutaryl phosphatidylethanolamine, or lysyl phosphatidylglycerol. In some cases, the non-cationic lipid may be, for example, distearoylphosphatidylcholine (DSPC), dioleoylphosphatidylcholine (DOPC), dipalmitoylphosphatidylcholine (DPPC), dioleoylphosphatidylglycerol (DOPG), dipalmitoylphosphatidylglycerol (DPPG), dioleoylphosphatidylethanolamine (DOPE), palmitoyloleoylphosphatidylcholine (POPC), palmitoyloleoylphosphatidylethanolamine (POPE), dioleoylphosphatidylethanolamine 4-(-maleimidomethyl)-cyclohexane-1-carboxylate (DOPE-mal), dipalmitoyl phosphatidyl ethanolamine (DPPE), dimyristoyl phosphoethanolamine (DMPE), distearoyl-phosphatidyl-ethanolamine (DSPE), 16-0-monomethyl PE, 16-O-dimethyl PE, 18-1-trans PE, 1-stearoyl-2-oleoyl-phosphatidylethanolamine (SOPE), cholesterol, phosphatidylglycerols, cardiolipins, diacyl phosphatidylserines, diacylphosphatidic acids, N-dodecanoyl phosphatidylethanolamines, N-succinyl phosphatidylethanolamines, N-glutarylphosphatidylethanolamines, lysyl phosphatidylglycerols, or palmitoyloleyolphosphatidylglycerol (POPG), but is not limited thereto.

The neutral lipid is specifically exemplified in U.S. Patent Publication Nos. 2018/0311176, 2019/0032051, etc., and, for example includes, but is not limited to, diacylphosphatidylcholine, diacylphosphatidylethanolamine, ceramide, sphingomyelin, dihydrosphingomyelin, cephalin, or cerebrosides.

A PEG lipid may be contained to prevent aggregation of particles produced during mRNA delivery. The PEG lipid is specifically exemplified in US Patent Publication Nos. 2018/0311176 and 2019/0032051, and is, for example, PEG-diacylglycerol (DAG), a PEG-dialkyloxypropyl (DAA), a PEG-phospholipid, a PEG-ceramide (Cer), or a mixture thereof. As a non-limiting example, PLGA may be conjugated to a lipid-terminating PEG forming PLGA-DSPE-PEG, PEG lipid is selected from PEG-c-DOMG, 1,2-dimyristoyl-sn-glycerol, methoxypolyethylene glycol (PEG-DMG), 1,2-distearoyl-sn-glycerol, methoxypolyethylene glycol (PEG-DSG), PEG-c-DOMG, 1,2-distearoyl-sn-glycerol, methoxypolyethylene glycol (PEG-DSG), 1,2-dipalmitoyl-sn-glycerol, methoxypolyethylene glycol (PEG-DPG), PEG-lipid conjugates such as, e.g., PEG coupled to dialkyloxypropyls (e.g., PEG-DAA conjugates), PEG coupled to diacylglycerols (e.g., PEG-DAG conjugates), PEG coupled to cholesterol, PEG coupled to phosphatidylethanolamines, PEG conjugated to ceramides, cationic PEG lipids, polyoxazoline (POZ)-lipid conjugates, polyamide oligomers (e.g., ATTA-lipid conjugates), and a mixture thereof. The PEG may be PEG-dilauryloxypropyl (C12), PEG-dimyristyloxypropyl (C14), PEG-dipalmityloxypropyl (C16), PEG-distearyloxypropyl (C18), PEG-c-DOMG, PEG-DMG, or a mixture thereof, but is not limited thereto.

Peptides may be used for mRNA delivery. The peptide must have a cation that electrostatically interacts with an anionic phosphate group of the nucleic acid and may contain a positively charged amino acid that electrostatically interacts with the phosphate group. Details of peptides that may be used for mRNA delivery are described in AIMS Biophysics, 7(4): 323-338, which is incorporated herein by reference.

The peptide that may be used for mRNA delivery may include protamine. Protamine is a small nuclear protein rich in cationic arginine that contributes to the stability of DNA during testicular spermatogenesis, which can stabilize mRNA molecules and enable efficient delivery. The protaminemRNA complex is described in detail in US Patent No. 9352028, which is incorporated herein by reference.

Cell-penetrating peptides (CPPs) may also be promising cationic molecules for delivery of mRNA. Amphipathic CPPs such as arginine-rich RALA peptide (WEARLARALARALARHLARALARALRACEA), RALA, LAH4 (KKALLALALHHLAHLALHLALALKKA), and LAH4-L1 (KKALLAHALHLLALLALHLAHALKKA) may be used to deliver mRNA molecules.

In some cases, peptides may be further used for mRNA delivery in addition to the liposome or LNP (lipid nanoparticle). The peptide functions to package nucleic acids and prevent DNA or RNA from being degraded intracellularly or extracellularly. Examples of such peptides are described in detail in US Patent Publication No. 2021/0170046, which is incorporated herein by reference, but are not limited thereto. The composition may further contain at least one pharmaceutically acceptable carrier. The pharmaceutically acceptable carrier must be compatible with the active ingredient of the present invention, may be saline solution, sterile water, Ringer's solution, buffered saline solution, dextrose solution, maltodextrin solution, glycerol, ethanol, or a mixture thereof, and may further contain other general additives such as antioxidants, buffers, and bacteriostatic agents as needed. In addition, the composition may be prepared into an injectable formulation such as an aqueous solution, suspension, or emulsion by adding a diluent, dispersant, surfactant, binder and lubricant thereto. In particular, the composition is preferably formulated into a lyophilizate. The lyophilizate may be prepared using a method commonly known in the art to which the present invention pertains and may be optionally prepared using a stabilizer for lyophilization. Furthermore, the composition is preferably formulated depending on each disease or ingredient using an appropriate method in the art or a method disclosed in Remington's Pharmaceutical Science (Mack Publishing company, Easton PA).

The content and administration method of the active ingredients contained in the composition of the present invention may be determined by those skilled in the art based on the severity of the symptoms and disease of the patient. In addition, the composition may be formulated in various forms such as powders, tablets, capsules, solutions, injections, ointments, and syrups, and may be provided in unit-dose or multi-dose containers, such as sealed ampoules and bottles.

The composition of the present invention may be administered orally or parenterally. The route of administration of the composition according to the present invention includes, but is not limited to, for example, bronchial, oral, intravenous, intramuscular, intraarterial, intramedullary, intrathecal, intracardiac, transdermal, subcutaneous, intraperitoneal, intestinal, sublingual or topical administration. The dosage of the composition according to the present invention varies depending on the weight, age, gender, health conditions, and diet of the patient, administration time, method, excretion rate, or severity of the disease, and is easily determined by those skilled in the art. In addition, the composition of the present invention may be formulated into a suitable dosage form for clinical administration using known techniques.

Hereinafter, the present invention will be described in more detail with reference to examples. However, it will be obvious to those skilled in the art that these examples are provided only for illustration of the present invention and should not be construed as limiting the scope of the present invention.

Example 1. cDNA synthesis for identification of 5'UTR and 3'UTR sequences of SARS-CoV-2 subgenomic RNA

To identify the 5' UTR and 3' UTR sequences of the subgenomic RNA of SARS-CoV-2, total RNA isolated from a culture of Vero cells infected with SARS-CoV-2 was obtained from Chungnam National University College of Veterinary Medicine (BSL3). To synthesize cDNA therefrom, 0.5 µg of oligo-dT was added to 1 µg of extracted total RNA to adjust a total amount to 12.5 ul, followed by allowing to react at 65°C for 5 minutes on ice. 4 µl of 5X reaction buffer, 1 mM dNTP mix, and 1 µl of RevertAid H minus reverse transcriptase (Enzynomics, Daejeon, South Korea) were added to the reaction solution to adjust a total amount of the reaction solution to 20 µl. A reverse transcription reaction was performed at 42°C for 60 minutes and 70°C for 10 minutes to synthesize single-stranded cDNA.

PCR was performed to amplify cDNA including the 5' UTR and 3' UTR of the N, S, and E genes of SARS-CoV-2 from the produced cDNA. The primer sequences used for PCR are set forth in Table 1.

**[Table 1] Primer sequences**

| Gene name | Forward primer (5'→3') | SEQ ID NO | Reverse Primer (5'→3') | SEQ ID NO |
|---|---|---|---|---|
| N | TAATACGACTCACTATAG GGATTAAAGGTTTATACC TTCCCAGG | 6 | TCTTCCTTGCCATGT TGAGT | 7 |
| S | TAATACGACTCACTATAG GGATTAAAGGTTTATACC TTCCCAGG | 6 | TCAAACCTCTTAGTA CCATTGG [?] | 8 |
| E | TAATACGACTCACTATAG GGATTAAAGGTTTATACC TTCCCAGG | 6 | AAGACTCACGTTAACA ATATTGC | 9 |

The PCR reaction was performed in a total of 20 µl solution, and 1 ng of synthesized cDNA, 10 pmole of a forward primer (SEQ ID NO: 7), 10 pmole of a reverse primer (SEQ ID NO: 7 to SEQ ID NO: 9), and 10 µl of 2X pfu Master Mix (Biofact, Daejeon, South Korea) were added. PCR reaction conditions were as follows: 95°C for 2 minutes (1 cycle); 95°C for 20 seconds - 60°C for 40 seconds - 72°C for 1 minute (30 cycles); 72°C (5 minutes). The PCR product was ligated into pTOP Blunt V2 vector (Enzynomics, Daejeon, South Korea) and then transformed into DH5α (Enzynomics, Daejeon, South Korea). After transformation, the result was spread on solid medium (Bioloard, Daejeon, South Korea) containing ampicillin (50 ug/ml, Sigma Aldrich, USA) and cultured at 37°C for 8 hours using HiGene^{™} Plasmid Mini Prep Kit (Ver.2.0) (Biofact, Daejeon, South Korea), plasmid DNA was extracted and Sanger sequencing was performed.

### Example 2. Identification of 5' UTR and common 3' UTR sequences of N, S, and E genes of SARS-CoV-2 through Sanger sequencing

**[Table 2] 5' UTR sequence**

| Gene name | 5' UTR sequence (5' →3' ) | SEQ ID NO |
|---|---|---|
| N | ATTAAAGGTTTATACCTTCCCAGGTAACAAACCAACC AACTTTCGATCTCTTGTAGATCTGTTCTCTAAACGAAC AAACTAAA | 2 |
| S | ATTAAAGGTTTATACCTTCCCAGGTAACAAACCAACC AACTTTCGATCTCTTGTAGATCTGTTCTCTAAACGAAC A | 3 |
| E | ATTAAAGGTTTATACCTTCCCAGGTAACAAACCAACC AACTTTCGATCTCTTGTAGATCTGTTCTCTAAACGAAC TT | 4 |

3'                                    UTR                                    5'-

CAATCTTTAATCAGTGTGTAACATTAGGGAGGACTTGAAAGAGCCACCACATTTTCACC

GAGGCCACGCGGAGTACGATCGAGTGTACAGTGAACAATGCTAGGGAGAGCTGCCTATA

TGGAAGAGCCCTAATGTGTAAAATTAATTTTAGTAGTGCTATCCCCATGTGATTTTAAT

AGCTTCTTAGGAGAATGAC-3' (SEQ ID NO: 5)

### Example 3. Construction of IVT template for expressing GFP mRNA

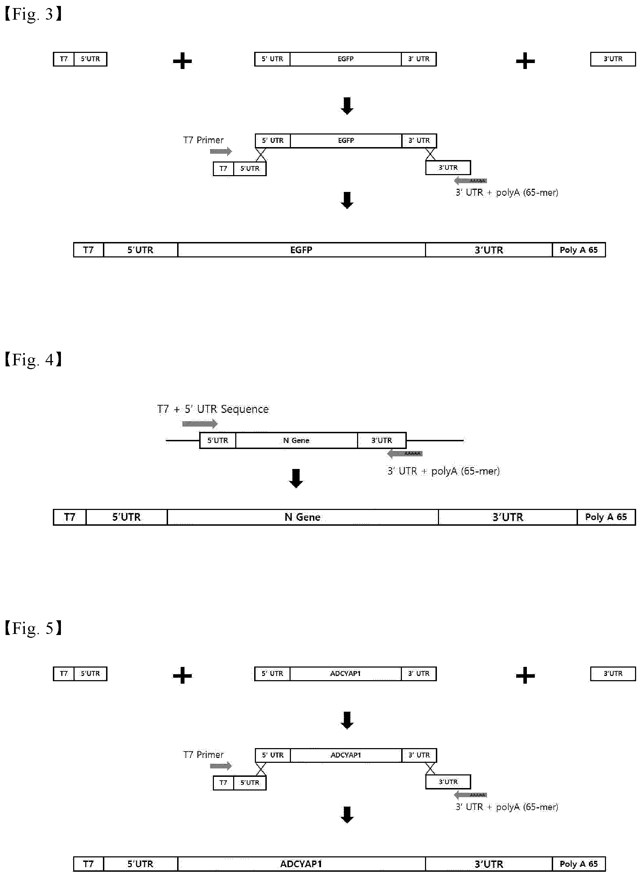

In order to construct an IVT template to express GFP mRNA, the GFP gene coding sequence was linked immediately after the 5' UTR of the N gene of SARS-CoV-2, and then 3' UTR sequence of the SARS-CoV-2 and the polyA sequence (65 nucleotides) were linked using PCR method (FIG. 3).

For this purpose, the T7 promoter sequence (SEQ ID NO: 10: 5'-TAATACGACTCACTATAGGG-3') was linked to the 5' UTR of the N gene by PCR. 10 pmole of a forward primer (SEQ ID NO: 6), 10 pmole of a reverse primer (SEQ ID NO: 11), and 10 µl of 2X pfu Master Mix (Biofact, Daejeon, South Korea) were added using 1 ng of plasmid DNA containing the N gene prepared in Example 1 as a template. PCR (Applied Biosystem) reaction conditions are as follows: 95°C for 2 minutes (1 cycle); 95°C for 20 seconds - 60°C for 40 seconds - 72°C for 1 minute (30 cycles); 72°C (5 minutes). The obtained PCR product was identified by electrophoresis on agarose gel (2%). 10 pmole of a forward primer (SEQ ID NO: 14) having a structure in which the 3' end sequence of the 5' UTR is linked to the 5' end sequence of the GFP gene, 10 pmole of a reverse primer (SEQ ID NO: 15) having a structure in which the 3' end sequence of the GFP gene is linked to the 5' end sequence of the 3' UTR, and 10 µl of 2X pfu Master Mix (Biofact, Daejeon, South Korea) were added using 1 ng of pEGFP N1 (Clontech) containing the GFP gene as a template. PCR (Applied Biosystem) reaction conditions were as follows: 95°C for 2 minutes (1 cycle); 95°C for 20 seconds - 60°C for 40 seconds - 72°C for 2 minutes (30 cycles); 72°C (5 minutes). The obtained PCR product was identified by electrophoresis on agarose gel (2%).

A PCR method was used to link the T7 promoter sequence, 5' UTR sequence, GFP gene sequence, 3' UTR sequence, and 65 nucleotide polyA sequence. A predetermined amount of 100 pg of each PCR product prepared above was mixed, and 10 pmole of a forward T7 promoter sequence primer (SEQ ID NO: 6), 10 pmole of a reverse primer (SEQ ID NO: 16) having a configuration in which the 20-nucleotide 3' end sequence of the 3' UTR is linked to the 65-nucleotide polyA sequence, and 10 µl of 2X pfu Master Mix (Biofact, Daejeon, South Korea) were added using the PCR product as a template. PCR (Applied Biosystem) reaction conditions were as follows: 95°C for 2 minutes (1 cycle); 95°C for 20 seconds - 60°C for 40 seconds - 72°C for 2 minutes (30 cycles); 72°C (for 5 minutes). The prepared PCR product was identified by electrophoresis on agarose gel (2%). The PCR product was ligated into pTOP Blunt V2 vector (Enzynomics, Daejeon, South Korea) and then transformed into DH5α (Enzynomics, Daejeon, South Korea). After transformation, the result was spread on solid medium (Bioloard, Daejeon, South Korea) containing ampicillin (50 ug/ml, Sigma Aldrich, USA) and cultured at 37°C for 8 hours with HiGene^{™} Plasmid Mini Prep Kit (Ver.2.0) (Biofact, Daejeon, South Korea), and then the IVT template sequence produced was identified by Sanger sequencing (SEQ ID NO: 18) .

To construct an IVT template for expression of control RNA containing no target gene, 10 pmole of a forward primer (SEQ ID NO: 16) having a structure in which the 3' end sequence of the 5' UTR is linked to the 5' end sequence of the 3' UTR, 10 pmole of a reverse primer (SEQ ID NO: 17) having a structure in which the 3' end sequence of the 3' UTR is linked to 65 nucleotides, and 10 µl of 2X pfu Master Mix (Biofact, Daejeon, South Korea) were added using 100 pg of the 3' UTR DNA prepared above as a template. PCR (Applied Biosystem) reaction conditions were as follows: 95°C for 2 minutes (1 cycle); at 95°C for 20 seconds - at 60°C for 40 seconds - at 72°C for 1 minute (30 cycles); 72°C (for 5 minutes). The obtained PCR product was identified by electrophoresis on agarose gel (2%).

A PCR method was used to link the T7 promoter sequence, 5' UTR sequence, 3' UTR sequence, and 65 nucleotide polyA sequence. A predetermined amount of 100 pg of each PCR product prepared above was mixed and 10 pmole of a forward T7 promoter sequence primer (SEQ ID NO: 6), 10 pmole of a reverse primer (SEQ ID NO: 16) having a configuration in which the 20-nucleotide 3' end sequence of the 3' UTR is linked to the 65-nucleotide polyA sequence, and 10 µl of 2X pfu Master Mix (Biofact, Daejeon, South Korea) were added using the PCR product as a template. PCR (Applied Biosystem) reaction conditions were as follows: 95°C for 2 minutes (1 cycle); 95°C for 20 seconds - 60°C for 40 seconds - 72°C for 2 minutes (30 cycles); 72°C (for 5 minutes). The prepared PCR product was identified by electrophoresis on agarose gel (2%) .

The PCR product was ligated into pTOP Blunt V2 vector (Enzynomics, Daejeon, South Korea) and then transformed into DH5α (Enzynomics, Daejeon, South Korea). After transformation, the result was spread on solid medium (Bioloard, Daejeon, South Korea) containing ampicillin (50 ug/ml, Sigma Aldrich, USA) and cultured at 37°C for 8 hours, plasmid DNA was extracted using HiGeneTM Plasmid Mini Prep Kit (Ver.2.0) (Biofact, Daejeon, South Korea), and the IVT template sequence produced was identified by Sanger sequencing (SEQ ID NO: 19).

**[Table 3] Primer sequences**

| Primer | Forward primer (5'→3') | SEQ ID NO |
|---|---|---|
| 5' UTR_R | TTTAGTTTGTTCGTTTAGAGAACAGATC | 11 |
| 3' UTR_F | CAATCTTTAATCAGTGTGTAACATTAGG | 12 |
| 3' UTR_R | GTCATTCTCCTAAGAAGCTATTAAAATCAC | 13 |
| 5' UTR+eGFP_F | CTCTAAACGAACAAACTAAAATGGTGAGCAAGGGCGAGGA | 14 |
| eGFP+3' UTR_R | TACACACTGATTAAAGATTGTTACTTGTACAGCTCGTCCA | 15 |
| 3' UTR (20) + Oligo dT (65)_R | TTTTTTTTTTTTTTTTTTTTTTTTTTTTTTTTTTTTTTTTTTTTTTTT TTTTTTTTTTTTTTTTTTTTTTTTGTCATTCTCCTAAGAAGCTATTAA | 16 |
| 5' UTR+3' UTR_F | CTCTAAACGAACAAACTAAACAATCTTTAATCAGTGTGTA | 17 |

[SEQ ID NO: 18] T7 5UTR eGFP 3' UTR oligo dT (65)

TAATACGACTCACTATAGGGATTAAAGGTTTATACCTTCCCAGGTAACAAACC
AACCAACTTTCGATCTCTTGTAGATCTGTTCTCTAAACGAACAAACTAAAATGGTGAGC
AAGGGCGAGGAGCTGTTCACCGGGGTGGTGCCCATCCTGGTCGAGCTGGACGGCGACGT
AAACGGCCACAAGTTCAGCGTGTCCGGCGAGGGCGAGGGCGATGCCACCTACGGCAAGC
TGACCCTGAAGTTCATCTGCACCACCGGCAAGCTGCCCGTGCCCTGGCCCACCCTCGTG
ACCACCCTGACCTACGGCGTGCAGTGCTTCAGCCGCTACCCCGACCACATGAAGCAGCA
CGACTTCTTCAAGTCCGCCATGCCCGAAGGCTACGTCCAGGAGCGCACCATCTTCTTCA
AGGACGACGGCAACTACAAGACCCGCGCCGAGGTGAAGTTCGAGGGCGACACCCTGGTG
AACCGCATCGAGCTGAAGGGCATCGACTTCAAGGAGGACGGCAACATCCTGGGGCACAA
GCTGGAGTACAACTACAACAGCCACAACGTCTATATCATGGCCGACAAGCAGAAGAACG
GCATCAAGGTGAACTTCAAGATCCGCCACAACATCGAGGACGGCAGCGTGCAGCTCGCC
GACCACTACCAGCAGAACACCCCCATCGGCGACGGCCCCGTGCTGCTGCCCGACAACCA
CTACCTGAGCACCCAGTCCGCCCTGAGCAAAGACCCCAACGAGAAGCGCGATCACATGG
TCCTGCTGGAGTTCGTGACCGCCGCCGGGATCACTCTCGGCATGGACGAGCTGTACAAG
TAACAATCTTTAATCAGTGTGTAACATTAGGGAGGACTTGAAAGAGCCACCACATTTTC
ACCGAGGCCACGCGGAGTACGATCGAGTGTACAGTGAACAATGCTAGGGAGAGCTGCCT
ATATGGAAGAGCCCTAATGTGTAAAATTAATTTTAGTAGTGCTATCCCCATGTGATTTT
AATAGCTTCTTAGGAGAATGACAAAAAAAAAAAAAAAAAAAAAAAAAAAAAAAAAAAAA
AAAAAAAAAAAAAAAAAAAAAAAAAAAAA

[SEQ ID NO: 19] T7 5' UTR 3' UTR oligodT (65)

TAATACGACTCACTATAGGGATTAAAGGTTTATACCTTCCCAGGTAACAAACCA
ACCAACTTTCGATCTCTTGTAGATCTGTTCTCTAAACGAACAAACTAAACAATCTTTAA
TCAGTGTGTAACATTAGGGAGGACTTGAAAGAGCCACCACATTTTCACCGAGGCCACGC
GGAGTACGATCGAGTGTACAGTGAACAATGCTAGGGAGAGCTGCCTATATGGAAGAGCC
CTAATGTGTAAAATTAATTTTAGTAGTGCTATCCCCATGTGATTTTAATAGCTTCTTAG

GAGAATGACAAAAAAAAAAAAAAAAAAAAAAAAAAAAAAAAAAAAAAAAAAAAAAAAAAAAAA

AAAAAAAAAAAAAAAA

### Example 4. Construction of IVT template for SARS-CoV-2 N gene mRNA expression

A PCR reaction was performed using 1 ng of cDNA synthesized in Example 1 in a total of 20 µl solution, and 10 pmole of a forward primer (SEQ ID NO: 6), 10 pmole of a reverse primer (SEQ ID NO: 16), and 10 µl of 2X pfu Master Mix (Biofact , Daejeon, South Korea) were added. PCR reaction conditions were as follows: 95°C for 2 minutes (1 cycle); 95°C for 20 seconds - 60°C for 40 seconds - 72°C for 2 minutes 30 seconds (30 cycles); 72°C (5 minutes). The PCR product was ligated into pTOP Blunt V2 vector (Enzynomics, Daejeon, South Korea) and then transformed into DH5α (Enzynomics, Daejeon, South Korea). After transformation, the result was spread on solid medium (Bioloard, Daejeon, South Korea) containing ampicillin (50 ug/ml, Sigma Aldrich, USA) and cultured at 37°C for 8 hours, plasmid DNA was extracted using HiGeneTM Plasmid Mini Prep Kit (Ver.2.0) (Biofact, Daejeon, South Korea), and then Sanger sequencing was performed (SEQ ID NO: 20).

### Example 5. Construction of IVT template for human ADCYAP1 gene mRNA expression

In order to construct an IVT template to express ADCYAP1 mRNA, the ADCYAP1 gene coding sequence was linked immediately after the 5' UTR of the N gene of SARS-CoV-2, and then 3' UTR sequence of the SARS-CoV-2 and the polyA sequence (65 nucleotides) were linked using PCR (FIG. 3).

For this purpose, 10 pmole of a forward primer (SEQ ID NO: 21) having a configuration in which the 3' end sequence of the 5' UTR is linked to the 5' end sequence of the ADCYAP1 gene, 10 pmole of a reverse primer (SEQ ID NO: 22) having a configuration in which the 3' end sequence of the ADCYAP1 gene is linked to the 5' end sequence of the 3' UTR, and 10 µl of 2X pfu Master Mix (Biofact, Daejeon, South Korea) were added using 1 ng of pcDNA3 plasmid DNA (Korean Patent No. 1399077) containing the ADCYAP1 gene as a template. PCR reaction conditions were as follows: 95°C for 2 minutes (1 cycle); 95°C for 20 seconds - 60°C for 40 seconds - 72°C for 2 minutes (30 cycles); 72°C (5 minutes).

A PCR method was used to link the T7 promoter sequence, 5' UTR sequence, ADCYAP1 gene sequence, 3' UTR sequence, and 65 nucleotide polyA sequence. A predetermined amount of 100 pg of each PCR product prepared above was mixed and 10 pmole of a forward T7 promoter sequence primer (SEQ ID NO: 6), 10 pmole of a reverse primer (SEQ ID NO: 16) having a configuration in which the 20-nucleotide 3' end sequence of the 3' UTR is linked to the 65-nucleotide polyA sequence, and 10 µl of 2X pfu Master Mix (Biofact, Daejeon, South Korea) were added using the PCR product as a template. PCR reaction conditions were as follows: 95°C for 2 minutes (1 cycle); 95°C for 20 seconds - 60°C for 40 seconds - 72°C for 2 minutes and 30 seconds (30 cycles); 72°C (for 5 minutes) . The prepared PCR product was identified by electrophoresis on agarose gel (2%). The PCR product was ligated into pTOP Blunt V2 vector (Enzynomics, Daejeon, South Korea) and then transformed into DH5α (Enzynomics, Daejeon, South Korea). After transformation, the result was spread on solid medium (Bioloard, Daejeon, South Korea) containing ampicillin (50 ug/ml, Sigma Aldrich, USA) and cultured at 37°C for 8 hours with HiGeneTM Plasmid Mini Prep Kit (Ver.2.0) (Biofact, Daejeon, South Korea), and then Sanger sequencing was performed (SEQ ID NO: 23).

**[Table 4] Primer sequences**

| Primer | Forward primer (5'→3') | SEQ ID NO |
|---|---|---|
| 5'UTR+ ADCYAP1_F | GCTCCGCTACACATGGTCATTTTAGTTTGTTCGTT TAGAG | 21 |
| ADCYAP1+3' UTR_R | GCCGAATAGCTTATTTGTAGCAATCTTTAATCAGT GTGTA | 22 |

[SEQ ID NO: 23] T7 5' UTR ADCYAP1 3' UTR oligo dT

(65)

TAATACGACTCACTATAGGGATTAAAGGTTTATACCTTCCCAGGTAACAAACCA
ACCAACTTTCGATCTCTTGTAGATCTGTTCTCTAAACGAACAAACTAAAATGACCATGT
GTAGCGGAGCGAGGCTGGCCCTGCTGGTCTATGGGATAATCATGCACAGCAGCGTCTAC
AGCTCACCTGCCGCCGCCGGACTCCGGTTCCCCGGGATCAGGCCAGAGGAAGAGGCGTA
CGGCGAGGACGGAAACCCGCTGCCAGACTTCGATGGCTCGGAGCCGCCGGGCGCAGGGA
GCCCCGCCTCCGCGCCGCGCGCCGCCGCCTGGTACCGCCCGGCCGGGAGAAGAGAT
GTCGCCCACGGGATCCTTAACGAGGCCTACCGCAAAGTGCTGGACCAGCTGTCCGCCGG
GAAGCACCTGCAGTCGCTCGTGGCCCGGGGCGTGGGTGGGAGCCTCGGCGGCGGCGCGG
GGGACGACGCGGAGCCGCTCTCCAAGCGCCACTCGGACGGGATCTTCACGGACAGCTAC
AGCCGCTACCGGAAACAAATGGCTGTCAAGAAATACTTGGCGGCCGTCCTAGGGAAGAG
GTATAAACAAAGGGTTAAAAACAAAGGACGCCGAATAGCTTATTTGTAGCAATCTTTAA
TCAGTGTGTAACATTAGGGAGGACTTGAAAGAGCCACCACATTTTCACCGAGGCCACGC
GGAGTACGATCGAGTGTACAGTGAACAATGCTAGGGAGAGCTGCCTATATGGAAGAGCC
CTAATGTGTAAAATTAATTTTAGTAGTGCTATCCCCATGTGATTTTAATAGCTTCTTAG
GAGAATGACAAAAAAAAAAAAAAAAAAAAAAAAAAAAAAAAAAAAAAAAAAAAAAAAAA
AAAAAAAAAAAAAAA

### Example 6. Identification of GFP, N gene, and ADCYAP1 mRNA expression through IVT

To produce each mRNA using the IVT method, 10 pmole of a forward primer corresponding to the T7 promoter sequence (SEQ ID NO: 6), 10 pmole of a reverse primer (SEQ ID NO: 16) having a configuration in which the 3' UTR is linked to 65-nucleotide polyA, and 10 µl of 2X pfu Master Mix (Biofact, Daejeon, South Korea) were added to adjust the total volume to 20 ul, followed by PCR. PCR reaction conditions were as follows: 95°C for 2 minutes (1 cycle); 95°C for 20 seconds - 60°C for 40 seconds - 72°C for 2 minutes 30 seconds (30 cycles); 72°C (5 minutes). The amplified PCR product was electrophoresed using a 1% agarose gel, purified using the Qiaquick^{®} Gel Extraction Kit (QIAGEN, Hilden, Germany), and used as a template for IVT.

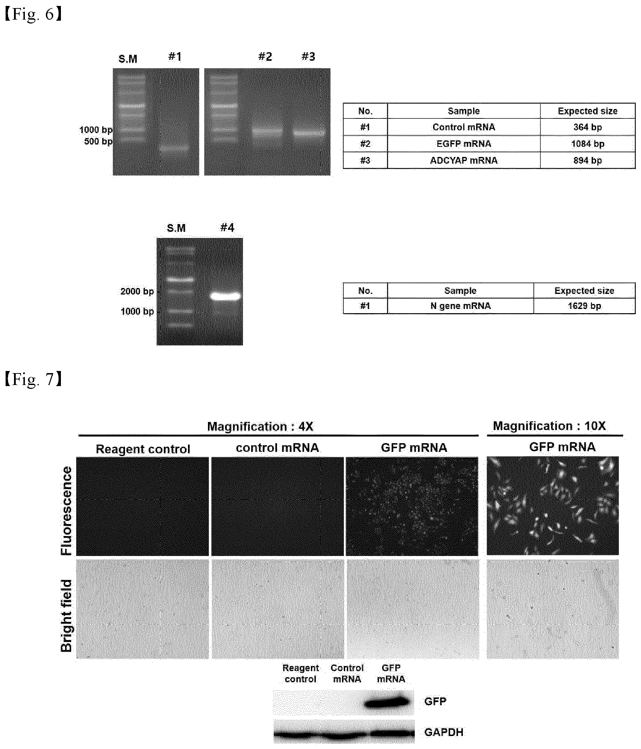

IVT was performed using Hiscribe T7 ARCA mRNA kit (NEB) in accordance with the manufacturer's instructions. Using 1 pg of the prepared PCR product as a template, 10 µl of 2X ARCA/NTP MIX and 2 µl of T7 polymerase were added and reacted with control at 37°C for 16 hours and reacted with each of GFP, ADCYAP, and N genes at 37°C for 1 hour to synthesize mRNA and further link an anti-reverse cap analog (ARCA) to the 5' end. After reaction, 2 µl of DNase I was added, followed by reaction at 37°C for 15 minutes to remove the DNA template. After the IVT reaction, the reaction solution was mixed with LiCl solution (NEB, Massachusetts, USA) (2:1 volume), followed by incubation at -20°C for 30 minutes, and then centrifugation at 13,000 rpm for 15 minutes to remove the supernatant. 500 µl of cold 70% ethanol was added, centrifuged at 13,000 rpm for 10 minutes to remove the supernatant, the residue was incubated at room temperature for 10 minutes to remove the remaining solution, and the solution was eluted in a total of 50 µl of RNasefree distilled water. The purified RNA was quantified using Qubit, and the mRNA product was identified by electrophoresis on agarose gel (1%, 0.5X TBE) (FIG. 6). The size of mRNA was measured after electrophoresis. As a result, the size matched the expected size.

### Example 7. Identification of GFP, N gene, and ADCYAP1 protein expression in human cell lines

### 7-1. Transfection and cell harvesting

The mRNA synthesized by the IVT method was mixed with Lipofectamine Messenger MAX (1:1, w:v), transfected into HeLa cell line, a cervical cancer cell line, and cultured in a CO₂ incubator for 48 hours. 48 hours after transfection, the expression of GFP was identified in the GFP mRNA-transfected cells through a fluorescence microscope and the cells were harvested. 48 hours after transfection, the ADCYAP1 mRNA-transfected cells were imaged, counted using a hematocytometer to measure cell viability and harvested. 48 hours after transfection, N gene mRNA-transfected cells were imaged and harvested. Proteins were extracted from the harvested cells using pro-prep (iNtRON biotechnology, Seongnam, South Korea) in accordance with the manufacturer's instructions.

### 7-2. Western blot

The extracted protein was subjected to SDS-PAGE using Mini-PROTEAN^{®} Tetra Vertical Electrophoresis Cell (Bio-rad, California, USA) and then transferred to a nitrocellulose membrane (Bio-rad, California, USA). The proteins transferred to the membrane were blocked with 5% skim milk (Biopure, Seoul, South Korea) for 1 hour and the membrane was added to a dilution of anti-GFP (Invitrogen, Massachusetts, USA), anti-ADCYAP1 (Santa Cruz Biotechnology, USA), anti-SARS-CoV-2 nucleocapsid (bioServUK, Sheffield, UK), and anti-GAPDH (Santa Cruz Biotechnology, Texas, USA) antibodies in 5% skim milk, followed by reaction at 4°C for 16 hours. The result was washed three times with 1X TBS-T for 10 minutes, reacted at room temperature for two hours in a dilution of goat anti-mouse IgG HRP and goat anti-rabbit IgG HRP (Santa Cruz Biotechnology, Texas, USA) as secondary antibodies in 5% skim milk, and washed three times with 1X TBS-T for 10 minutes. The membrane was treated with a mixture of WesternBright Peroxide solution (Advansta, California, USA) and WesternBright ECL solution (Advansta, California, USA) in a ratio of 1:1, and reacted at room temperature for 1 minute, and then protein expression was identified using Chemidoc XRS+ (Bio-rad, California, USA) equipment.

### 7-3. Identification of GFP protein expression through fluorescence imaging and Western blot.

48 hours after transfection into the HeLa cell line, the cells were observed through a fluorescence microscope. The results show that expression of GFP could not be identified in cells treated with reagents and control mRNA, but expression of GFP could be identified in cells treated with GFP mRNA. Cells were harvested, protein was extracted, and western blot was performed. The result shows that GFP expression was identified only in cells treated with GFP mRNA (FIG. 7). This indicates that the mRNA produced in the present invention using the 5' UTR and 3' UTR sequences of SARS-CoV-2 is expressed well as proteins in cells. Therefore, this suggests that other mRNAs can be expressed according to the present invention.

### 7-4. Identification of cell proliferation inhibitory effect of ADCYAP1 and identification of ADCYAP1 protein expression through Western blot

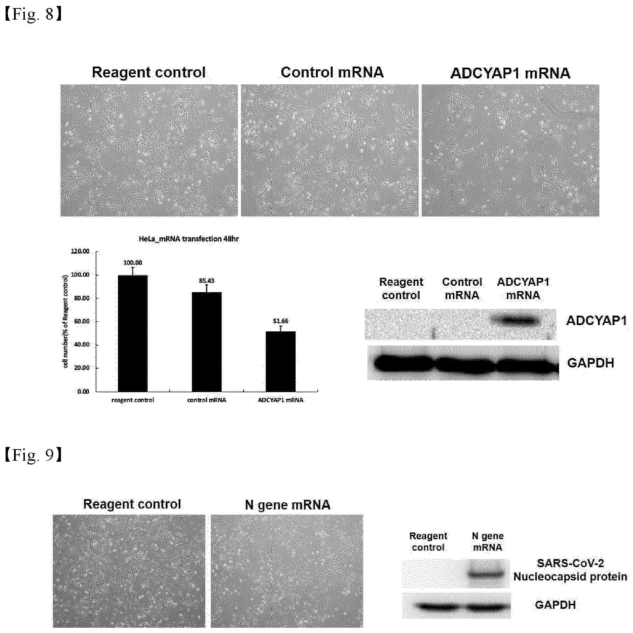

ADCYAP1 is known to inhibit the growth of cervical cancer cells (Korean Patent No. 1399077). 48 hours after transfection into the cervical cancer HeLa cell line, cell growth was observed. The result shows that the cells transfected with ADCYAP mRNA decreased by about 40% compared to cells transfected with control mRNA (FIG. 8). In addition, cells were harvested, protein was extracted, and Western blot was performed. The result shows that ADCYAP1 protein expression increased in cells treated with ADCYAP1 mRNA. This indicates that cell proliferation was inhibited by the ADCYAP1 protein expressed within the cells (FIG. 8). These results suggest that the mRNA produced according to the present invention can be used for cancer treatment.

### 7-5. Identification of nucleocapsid protein expression through Western blot

48 hours after transfection into the HeLa cell line, cells were harvested, protein extracted, and Western blot was performed. The result shows that SARS-CoV-2 nucleocapsid protein was expressed in cells treated with N gene mRNA (FIG. 9) .

Although specific configurations of the present invention have been described in detail, those skilled in the art will appreciate that this detailed description is provided as preferred embodiments for illustrative purposes and should not be construed as limiting the scope of the present invention. Therefore, the substantial scope of the present invention is defined by the accompanying filed claims and equivalents thereto.

### [Industrial applicability]

The gene construct according to the present invention allows expression of mRNA that can provide increased protein production. This provides a pharmaceutical composition that can be used for vaccines or gene therapy.

### [Sequence Listing Free Text]

An electronic file is attached.

## Claims

1. A gene construct comprising:
a coding region for expressing mRNA of a target gene;
a 5' untranslated region (UTR) located upstream of the coding region; and
a 3' untranslated region (UTR) located downstream of the coding region,
wherein the 5' untranslated region (UTR) comprises a leader sequence of a coronavirus.

2. The gene construct according to claim 1, wherein the 5' untranslated region (UTR) comprises a leader sequence of the coronavirus (SARS-CoV-2: severe acute respiratory syndrome coronavirus 2) represented by SEQ ID NO: 1.

3. The gene construct according to claim 1, wherein the 5' untranslated region (UTR) is a gene construct comprising a leader sequence from human coronavirus 229E, OC43, HKU1, NL63, SARS-1, or MERS.

4. The gene construct according to claim 1, wherein the 5' untranslated region (UTR) comprises at least one sequence selected from the group consisting of SEQ ID NO: 2 to SEQ ID NO: 4.

5. The gene construct according to claim 1, wherein the 3' untranslated region (UTR) comprises a sequence of SEQ ID NO: 5.

6. The gene construct according to claim 1, further comprising a promoter and/or poly(A) sequence.

7. The gene construct according to claim 6, wherein the promoter is located upstream of the 5' untranslated region (UTR).

8. The gene construct according to claim 6, wherein the poly(A) sequence is located downstream of the 3' untranslated region (UTR).

9. The gene construct according to claim 1, further comprising a sequence of SEQ ID NO: 18 or 19.

10. A vector comprising the gene construct according to any one of claims 1 to 9.

11. A pharmaceutical composition comprising the gene construct according to any one of claims 1 to 9.

12. A vaccine composition comprising the gene construct according to any one of claims 1 to 9.

13. A composition for gene therapy comprising the gene construct according to any one of claims 1 to 9.
